# EUROPEAN PATENT APPLICATION

(11) **EP 4 779 024 A2**
(43) Date of publication of application: **22.07.2026**
(21) Application number: 26180634.3
(22) Date of filing: 26.04.2019
(51) Int. Cl.: C12N 15/864

(54) **MODIFIED AAV CAPSID POLYPEPTIDES FOR TREATMENT OF MUSCULAR DISEASES**

(30) Priority: 27.04.2018 EP 18169822
(62) Divisional of application: 19722029.6
(71) Applicant: Universität Heidelberg, 69117 Heidelberg (DE)
(72) Inventor: Grimm, Dirk, 69117 Heidelberg (DE); Weinmann, Jonas, 4056 Basel (CH); El Andari, Josef, 69115 Heidelberg (DE)
(74) Representative: Altmann Stößel Dick Patentanwälte PartG mbB

(57) **Abstract**

The present invention relates to an adeno-associated virus (AAV) capsid polypeptide bonded to a binding peptide comprising an amino acid sequence RGDX¹X²X³X⁴, with X¹ to X⁴ being independently selected amino acids, for use in treating and/or preventing a muscular disease and/or in muscle regeneration. The present invention further relates to polynucleotides, host cells, adeno-associated virus (AAV) capsids, pharmaceutical compositions, uses, and methods related to said AAV capsid polypeptide.

## Description

The present invention relates to an adeno-associated virus (AAV) capsid polypeptide bonded to a binding peptide comprising an amino acid sequence RGDX¹X²X³X⁴ (SEQ ID NO: 1), with X¹ to X⁴ being independently selected amino acids, for use in treating and/or preventing a muscular disease and/or in muscle regeneration. The present invention further relates to polynucleotides, host cells, adeno-associated virus (AAV) capsids, pharmaceutical compositions, uses, and methods related to said AAV capsid polypeptide.

Adeno-associated virus (AAV) vectors have been known to enable transfer and long-term expression of foreign DNA, including therapeutic genes, in a variety of cell types. The different serotypes of AAV have been shown to have different tropisms in cultured cells and within an organism, with AAV9 e.g. having the broadest tropism. Transduction efficiencies and cell specificities obtainable with AAV vectors comprising unmodified capsids are good for some cell types, however, they are far too low to be of experimental or therapeutic use for most other cell types. A further problem in retargeting AAV is that while it may be possible to confer a new specificity to an AAV strain by including amino acid exchanges and/or additional peptides into the capsid protein, frequently a substantial infectivity for the liver, the major target of AAV, remains, which is undesirable for most applications.

Over the last decade many novel synthetic AAV variants have emerged by using a variety of capsid engineering techniques, one of which is the insertion of small, 7 amino acid-long, peptides into an exposed loop of the capsid protein, called variable region VIII (VRVIII). The implementation of a novel peptide into a wild type capsid has shown to be very promising for permanently changing the tropism of the variant. Insertion of a peptide P1 (RGDLGLS) into the capsid gene of AAV9 was found to increase infection of astrocytes (see PhD thesis of Eike Kienle, Ruprecht-Karls-Universität Heidelberg, 2014) and primary breast cancer cells (Michelfelder et al. (2009)).

One desirable target for AAV infection are muscle cells. A variety of AAV variants has been provided with improved infectivity for these cells (Yu et al (2009), Choudhury et al (2016), and Yang et al (2009)); nonetheless, there is still a need in the art for improved AAV vectors targeting these cells. It is therefore an objective of the present invention to provide means and methods to comply with the aforementioned needs, avoiding at least in part the shortcomings of the prior art. This problem is solved by compounds, methods, and uses of the present invention. Embodiments, which might be realized in an isolated fashion or in any arbitrary combination, are listed in the dependent claims.

Accordingly, the present invention relates to an adeno-associated virus (AAV) capsid polypeptide bonded to a binding peptide comprising an amino acid sequence RGDX¹X²X³X⁴ (SEQ ID NO: 1), with X¹ to X⁴ being independently selected amino acids, for use in treating and/or preventing a muscular disease and/or in muscle regeneration.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

As used herein, the term "standard conditions", if not otherwise noted, relates to IUPAC standard ambient temperature and pressure (SATP) conditions, i.e. preferably, a temperature of 25°C and an absolute pressure of 100 kPa; also preferably, standard conditions include a pH of 7. Moreover, if not otherwise indicated, the term "about" relates to the indicated value with the commonly accepted technical precision in the relevant field, preferably relates to the indicated value ± 20%, more preferably ± 10%, most preferably ± 5%. Further, the term "essentially" indicates that deviations having influence on the indicated result or use are absent, i.e. potential deviations do not cause the indicated result to deviate by more than ± 20%, more preferably ± 10%, most preferably ± 5%. Thus, "consisting essentially of" means including the components specified but excluding other components except for materials present as impurities, unavoidable materials present as a result of processes used to provide the components, and components added for a purpose other than achieving the technical effect of the invention. For example, a composition defined using the phrase "consisting essentially of" encompasses any known acceptable additive, excipient, diluent, carrier, and the like. Preferably, a composition consisting essentially of a set of components will comprise less than 5% by weight, more preferably less than 3% by weight, even more preferably less than 1%, most preferably less than 0.1% by weight of non-specified component(s). In the context of nucleic acid sequences, the term "essentially identical" indicates a %identity value of at least 80%, preferably at least 90%, more preferably at least 98%, most preferably at least 99%. As will be understood, the term essentially identical includes 100% identity. The aforesaid applies to the term "essentially complementary" mutatis mutandis. For the avoidance of doubt, an expression referring to a compound in the singular (e.g. "a capsid") may refer to a single instance of said compound, but also to a multitude of instances of said compound.

As used herein, the term "Adeno-Associated Virus" or "AAV" relates to the group of viruses containing a short (approx. 4.7 kB) single-stranded DNA and depending in their lytic replication on the presence of an Adenovirus. AAVs are members of the Parvoviridae family of viruses. Also contemplated by the present invention are vectors derived from AAV, i.e. gene transfer vehicles using the capsid polypeptide of AAV to mediate the transfer of recombinant polynucleotides into target cells. The term "AAV capsid polypeptide" as meant herein relates to a polypeptide with the activity of self-assembly to produce the proteinaceous shell of an AAV particle, also referred to as coat protein or VP protein. It is to be understood that not all AAV capsid polypeptide molecules in a given cell assemble into AAV capsids. Preferably, at least 25%, at least 50%, at least 75%, at least 85%, at least 90%, at least 95% of all AAV capsid polypeptide molecules assemble into AAV capsids. Suitable assays for measuring this biological activity are described e.g. in Smith-Arica and Bartlett (2001), Curr Cardiol Rep 3(1): 43-49. Preferably, the AAV capsid polypeptide is the capsid polypeptide of AAV9 (Genbank Acc. No: AAS99264.1), AAV1 (Genbank Acc. No: AAD27757.1), AAV2 (Genbank Acc. No: AAC03780.1), AAV3 (Genbank Acc. No: AAC55049.1), AAV3b (Genbank Acc. No: AF028705.1), AAV4 (Genbank Acc. No: AAC58045.1), AAV5 (Genbank Acc. No: AAD13756.1), AAV6 (Genbank Acc. No: AF028704.1), AAV7 (Genbank Acc. No: AAN03855.1), AAV8 (Genbank Acc. No: AAN03857.1), AAV10 (Genbank Acc. No: AAT46337.1), AAVrh10 (Genbank Acc. No: AY243015.1), AAV11 (Genbank Acc. No: AAT46339.1), AAV12 (Genbank Acc. No: ABI16639.1), or AAV13 (Genbank Acc. No: ABZ10812.1), AAVpol (Genbank Acc. No: FJ688147.1). More preferably, the AAV capsid polypeptide is the capsid polypeptide of AAV9 (Genbank Acc. No: AAS99264.1).

The term AAV capsid polypeptide, as used herein, preferably includes polypeptide variants of said capsid polypeptides. As used herein, the term "polypeptide variant" relates to any chemical molecule comprising at least one polypeptide or fusion polypeptide as specified elsewhere herein, having the indicated activity, but differing in primary structure from said polypeptide or fusion polypeptide indicated above. Thus, the polypeptide variant, preferably, is a mutein having the indicated activity. Preferably, the polypeptide variant comprises a peptide having an amino acid sequence corresponding to an amino acid sequence of 500 to 1000, more preferably 650 to 800, even more preferably 700 to 770, or, most preferably, 710 to 750 consecutive amino acids comprised in a polypeptide as specified above. Moreover, also encompassed are further polypeptide variants of the aforementioned polypeptides. Such polypeptide variants have at least essentially the same biological activity as the specific polypeptides. Moreover, it is to be understood that a polypeptide variant as referred to in accordance with the present invention shall have an amino acid sequence which differs due to at least one amino acid substitution, deletion and/or addition, wherein the amino acid sequence of the variant is still, preferably, at least 50%, 60%, 70%, 80%, 85%, 90%, 92%, 95%, 97%, 98%, or 99% identical with the amino acid sequence of the specific polypeptide. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the sequence it is compared to for optimal alignment. The percentage is calculated by determining, preferably over the whole length of the polypeptide, the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman (1981), by the homology alignment algorithm of Needleman and Wunsch (1970), by the search for similarity method of Pearson and Lipman (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Polypeptide variants referred to herein may be allelic variants or any other species specific homologs, paralogs, or orthologs. Moreover, the polypeptide variants referred to herein include fragments of the specific polypeptides or the aforementioned types of polypeptide variants as long as these fragments and/or variants have the biological activity as referred to above. Such fragments may be or be derived from, e.g., degradation products or splice variants of the polypeptides. Further included are variants which differ due to posttranslational modifications such as phosphorylation, glycosylation, ubiquitinylation, sumoylation, or myristylation, by including non-natural amino acids, and/or by being peptidomimetics.

Preferably, the AAV capsid polypeptide variant is a chimeric capsid polypeptide. The term "chimeric" is understood by the skilled person to relate to any molecule composed of components originating from at least two different species or strains. Thus, preferably, the capsid polypeptide variant comprises amino acid sequences derived from capsid polypeptides of at least two, preferably at least three AAV strains, more preferably of the AAV strains as specified elsewhere herein. More preferably, the capsid polypeptide variant comprises amino acid sequences of at least five, preferably at least ten, more preferably at least 20 contiguous amino acids derived from capsid polypeptides of at least two, preferably at least three AAV strains. Preferably, the polynucleotide encoding said chimeric capsid polypeptide was generated by DNA shuffling, a technique which is, in principle, known to the skilled person.

The term "binding peptide", as used herein, relates to a peptide comprising, preferably consisting of, a sequence RGDX¹X²X³X⁴ (SEQ ID NO: 1), with X¹ to X⁴ being independently selected amino acids (SEQ ID NO:1). As used herein, the term "amino acid" relates to α-aminocarboxylic acids, including artificial, i.e. non-naturally occurring, α-amino acids. Preferably, the amino acid is a naturally occurring amino acid. Preferably, the amino acid is an L-α-amino acid, more preferably a naturally occurring L-α-amino acid, most preferably is a proteinogenic amino acid, i.e., preferably, an amino acid naturally incorporated into a polypeptide during protein biosynthesis. Preferably, in the binding peptide according to SEQ ID NO:1, X¹, X², and X³ are independently selected from L, G, V, and A; and X⁴ is S, V, A, G, or L. In a preferred embodiment, X¹ is selected from L, Q, D, H, M, P, and K, preferably is L; X² is selected from G, V, S, D, M, and N, preferably is G; X³ is selected from V, M, P, S, and D, preferably is V; and/or X⁴ is selected from S, N, L, H, and M, preferably is S. In a further preferred embodiment, in the binding peptide according to SEQ ID NO:1, X¹ is L. In a further preferred embodiment, in the binding peptide according to SEQ ID NO:1, X² is G. In a further preferred embodiment, in the binding peptide according to SEQ ID NO:1, X⁴ is S. In a further preferred embodiment, X¹ is L; X² is selected from G and V, preferably is G; X³ is V; and/or X⁴ is selected from S and L, preferably is S. In a further preferred embodiment, in the binding peptide according to SEQ ID NO:1, X¹ is L, X² is G, and/or X⁴ is S. More preferably, in the binding peptide according to SEQ ID NO:1, at least one of X² and X³ is G. Even more preferably, the binding peptide comprises, preferably consists of, the amino acid sequence RGDLGLS (SEQ ID NO:2), RGDAVGV (SEQ ID NO:3), and/or a sequence comprising at most two, preferably at most one amino acid substitution compared to one of the aforesaid specific sequences. Still more preferably, the binding peptide comprises, preferably consists of, the amino acid sequence RGDLGLS (SEQ ID NO:2) and/or RGDAVGV (SEQ ID NO:3). Still more preferably, the binding peptide comprises, preferably consists of, the amino acid sequence RGDLGLS (SEQ ID NO:2) or the binding peptide comprises, preferably consists of, the amino acid sequence RGDAVGV (SEQ ID NO:3). Still more preferably, the binding peptide comprises, preferably consists of, the amino acid sequence RGDLGLS (SEQ ID NO:2) and/or a sequence comprising at most two, preferably at most one amino acid substitution compared to said sequence. Most preferably, the binding peptide comprises, preferably consists of, the amino acid sequence RGDLGLS (SEQ ID NO:2).

The binding peptide is bonded to the AAV capsid polypeptide. As used herein, the expression "is bonded" relates to a chemical association between the binding peptide and the AAV capsid polypeptide having a dissociation constant K_{d} of at most 10⁻⁵ mol/l (as it is the case with the Strep-Tag:Strep-Tactin binding). Preferably, said K_{d} is at most 10⁻⁶ mol/l (as it is the case in the Strep-TagII:Strep-Tactin binding), more preferably at most 10⁻⁷ mol/l (as it is typically the case in antibody:antigen binding), still more preferably at most 10⁻⁸ mol/l, most preferably at most 10⁻¹⁰ mol/l (as it is the case for the Streptavidin:Biotin binding). E.g., preferably, biotin-conjugated binding peptides may be used for bonding to streptavidin-conjugated AAV capsid polypeptides. Methods of determining dissociation constants are well known to the skilled artisan and include, e.g., spectroscopic titration methods, surface plasmon resonance measurements, equilibrium dialysis and the like.

Preferably, the binding peptide is bonded covalently to the AAV capsid polypeptide; thus, preferably, there is at least one covalent chemical bond connecting the binding peptide to the AAV capsid polypeptide. As will be understood, the covalent bonding may be indirect, e.g. via the atoms of a linker molecule. Preferably, the chain of atoms connecting the binding peptide to the AAV capsid polypeptide comprises at most 20 covalent bonds, more preferably at most ten covalent bonds, still more preferably at most five covalent bonds. Most preferably, the binding peptide is bonded directly to the AAV capsid polypeptide.

Preferably, the binding peptide is inserted into the AAV capsid polypeptide, i.e., preferably, the binding peptide has a direct covalent bond to the AAV capsid polypeptide at its N-terminus and a direct covalent bond to the AAV capsid polypeptide at its C-terminus, interrupting the amino acid sequence of the AAV capsid polypeptide. Thus, preferably, the binding peptide and the AAV capsid polypeptide are continuous in sequence. Accordingly, the present invention also relates to an adeno-associated virus (AAV) capsid polypeptide comprising an inserted binding peptide comprising an amino acid sequence RGDX¹X²X³X⁴ (SEQ ID NO: 1), as specified above. Preferably, the binding peptide is inserted at a site of the capsid polypeptide exposed to the exterior of the AAV capsid, preferably based on structure predictions and/or experimental data. More preferably, the insertion site of the binding peptide is at a site exposed to the exterior of the AAV capsid in a manner that does not interfere with the activity of said polypeptide in capsid assembly. More preferably, the insertion site of the binding peptide corresponds to amino acid 588 or 589, preferably 588, of the AAV9 capsid polypeptide. Whether an insertion site in an AAV capsid polypeptide corresponds to an insertion site in the AAV9 capsid polypeptide can be established by the skilled person by known methods, preferably by aligning the amino acids of the capsid polypeptides, preferably as specified elsewhere herein. In preferred embodiments, the insertions sites correspond to the amino acids and optionally have the modified flanking amino acids as shown in Table 1. As will be understood by the skilled person from Table 1, an insertion site of the binding peptide corresponding to amino acid 588 or 589, preferably 588, of the AAV9 capsid polypeptide, preferably is an insertion site at amino acid 567 to 594 of the actual AAV capsid polypeptide. Thus, preferred insertion sites are 588 or 589, more preferably 588 in AAV9, and 587, 588, or 589 in chimeric AAV polypeptides, in particular those used in SEQ ID NOs: 28, 30, 32, and 34. In a preferred embodiment, the sequence of SEQ ID NO:1 is flanked N-terminally by the amino acids S or R and G, i.e. by SG or RG; and C-terminally by an A in the capsid polypeptide.

**Table 1: Preferred insertion site/flanking amino acids combinations. Amino acids between which insertion of a peptide is preferred are separated by a "-"; Position indications are relative to the amino acid sequence of the respective wildtype capsid polypeptide as indicated elsewhere herein.**

| | insertion sites 1: | | insertion sites 2: | |
|---|---|---|---|---|
| Serotype | Insertion Site | Modified Amino Acids (SEQ ID NO:) | Insertion Site | Modified Amino Acids (SEQ ID NO:) |
| AAV1 | D590_P591 | STD-PAT (36) | S588_T589 | SSS-TDP (37) |
| AAV2 | R588_Q589 | GNR-QAA (38) | N587_R588 | RGN-RQA (39) |
| AAV3b | S586_S587 | LQS-SNT (40) | N588_T589 | S SN-TAP (41) |
| AAV4 | S584_N585 | DQS-NSN (42) | S586_N587 | SNS-NLP (43) |
| AAV5 | S575_S576 | NQS-STT (44) | T577_T578 | SST-TAP (45) |
| AAV6 | D590_P591 | STD-PAT (46) | S588_T589 | SSS-TDP (47) |
| AAV7 | N589_T590 | AAN-TAA (48) | - | - |
| AAV8 | N590_T591 | QQN-TAP (49) | - | - |
| AAV9 | Q588_A589 | SAQ-AQA (50) | - | - |
| AAVrh10 | N590_A591 | QQN-AAP (51) | - | - |
| AAVpo.1 | N567_S568 | NQN-SNT (52) | N569_T570 | NSN-THP (53) |
| AAV12 | N592_A593 | NQN-ATT (54) | T594_T595 | NAT-TAP (55) |

Most preferably, the AAV capsid polypeptide is an AAV9 capsid polypeptide and the insertion site of the binding peptide is amino acid 588 or 589, preferably 588. For the avoidance of doubt, as used herein, the indication that an insertion site is at amino acid X means that the binding peptide is inserted between amino acids X and X+1, i.e., preferably, the binding peptide is inserted after the indicated amino acid.

Preferably, the AAV capsid polypeptide comprising an inserted binding peptide comprises, more preferably consists of, the amino acid sequence of SEQ ID NO: 12, preferably encoded by a polynucleotide comprising SEQ ID NO:13; the amino acid sequence of SEQ ID NO:14, preferably encoded by a polynucleotide comprising SEQ ID NO:15; the amino acid sequence of SEQ ID NO:16, preferably encoded by a polynucleotide comprising SEQ ID NO:17; the amino acid sequence of SEQ ID NO: 18, preferably encoded by a polynucleotide comprising SEQ ID NO:19; the amino acid sequence of SEQ ID NO:20, preferably encoded by a polynucleotide comprising SEQ ID NO:21; the amino acid sequence of SEQ ID NO:22, preferably encoded by a polynucleotide comprising SEQ ID NO:23; the amino acid sequence of SEQ ID NO:24, preferably encoded by a polynucleotide comprising SEQ ID NO:25; or the amino acid sequence of SEQ ID NO:26, preferably encoded by a polynucleotide comprising SEQ ID NO:27. Also preferably, the (chimeric) AAV capsid polypeptide comprising an inserted binding peptide comprises, more preferably consists of, the amino acid sequence of SEQ ID NO:28, preferably encoded by a polynucleotide comprising SEQ ID NO:29; the amino acid sequence of SEQ ID NO:30, preferably encoded by a polynucleotide comprising SEQ ID NO: 31; the amino acid sequence of SEQ ID NO:32, preferably encoded by a polynucleotide comprising SEQ ID NO: 33; or the amino acid sequence of SEQ ID NO:34, preferably encoded by a polynucleotide comprising SEQ ID NO: 35. More preferably, the AAV capsid polypeptide comprises, more preferably consists of, the amino acid sequence of SEQ ID NO:12 or 28.

According to the present invention, the AAV capsid polypeptide preferably comprises or further comprises an amino acid exchange corresponding to a P504A and/or a G505A amino acid exchange in AAV9. Whether amino acids correspond in two non-identical AAV capsid polypeptides can be established by methods known to the skilled person and as described elsewhere herein, in particular by alignment. An amino acid exchange corresponding to an exchange for an A, preferably, is an exchange for an A, G, or V, more preferably is an exchange for an A. Preferably, the AAV capsid polypeptide is an AAV9 capsid polypeptide and the AAV capsid polypeptide comprises or further comprises a P504A and/or a G505A amino acid exchange, preferably comprises or further comprises a P504A and G505A amino acid exchange.

The term "treatment" refers to an amelioration of a disease or disorder referred to herein or the symptoms accompanied therewith to a significant extent. Said treating as used herein also includes an entire restoration of the health with respect to the diseases or disorders referred to herein. It is to be understood that treating as used in accordance with the present invention may not be effective in all subjects to be treated. However, the term shall require that, preferably, a statistically significant portion of subjects suffering from a disease or disorder referred to herein can be successfully treated. Whether a portion is statistically significant can be determined without further ado by the person skilled in the art using various well known statistic evaluation tools, e.g., determination of confidence intervals, p-value determination, Student's t-test, Mann-Whitney test etc.. Preferred confidence intervals are at least 90%, at least 95%, at least 97%, at least 98% or at least 99%. The p-values are, preferably, 0.1, 0.05, 0.01, 0.005, or 0.0001. Preferably, the treatment shall be effective for at least 20%, more preferably at least 50%, even more preferably at least 60%, still more preferably at least 70%, still more preferably at least 80%, most preferably at least 90% of the subjects of a given cohort or population. As will be understood by the skilled person, effectiveness of treatment is dependent on a variety of factors including, e.g. disease stage and severity. As will be understood, the term treatment includes measures preventing and/or delaying progression of a disease or disorder referred to herein.

The tem "preventing" refers to retaining health with respect to the diseases or disorders referred to herein for a certain period of time in a subject. It will be understood that the said period of time is dependent on the amount of the drug compound which has been administered and individual factors of the subject discussed elsewhere in this specification. It is to be understood that prevention may not be effective in all subjects treated with the compound according to the present invention. However, the term requires that a statistically significant portion of subjects of a cohort or population are effectively prevented from suffering from a disease or disorder referred to herein or its accompanying symptoms. Preferably, a cohort or population of subjects is envisaged in this context which normally, i.e. without preventive measures according to the present invention, would develop a disease or disorder as referred to herein. Whether a portion is statistically significant can be determined without further ado by the person skilled in the art using various well known statistic evaluation tools discussed elsewhere in this specification. As used herein, the term prevention, preferably, includes measures of gene therapy, as described elsewhere herein. Thus, the subject receiving preventive measures may be free of any symptoms of disease, but may be identified e.g. by gene analysis to have an increased risk of developing a disease or disorder referred to herein.

The term "muscle" is understood by the skilled person. Preferably, the muscle is a muscle of a vertebrate, more preferably of a mammal, even more preferably of an animal, most preferably of a human subject. Preferably, the muscle is a smooth muscle or a striated muscle, more preferably a striated muscle. Preferably, the muscle is a heart muscle, a skeletal muscle, or a muscle of the diaphragm. Thus, preferably, a "subject", as used herein, is a vertebrate, more preferably a mammal, even more preferably a livestock or companion animal, such as a chicken, a goose, a duck, a goat, a sheep, a cattle, a pig, a horse, a dog, a cat, a hamster, a rat, a mouse, a hamster, or a guinea pig. Most preferably, the subject is a human.

The term "muscular disease" is, in principle, understood by the skilled person. Preferably, the term relates to a disease amenable to treatment and/or prevention by administration of an active compound to a muscle, in particular to a muscle cell. Preferably, the muscular disease is a muscular dystrophy, a cardiomyopathy, a myotonia, a muscular atrophy, a myoclonus dystonia (affected gene: SGCE), a mitochondrial myopathy, a rhabdomyolysis, a fibromyalgia, and/or a myofascial pain syndrome.

Preferably, the muscular dystrophy is Duchenne muscular dystrophy (gene affected: DMD), Becker muscular dystrophy (gene affected: DMD), Limb girdle muscular dystrophy (Subtypes and affected genes: LGMD1A (Gene: TTID), LGMD1B (Gene: LMNA), LGMD1C (Gene: CAV3), LGMD1D (Gene: DNAJB6), LGMD1E (Gene: DES), LGMD1F (Gene: TNPO3), LGMD1G (Gene: HNRPDL), LGMD1H, LGMD2A (Gene: CAPN3), LGMD2B (Gene: DYSF), LGMD2C (Gene: SGCG), LGMD2D (Gene: SGCA), LGMD2E (Gene: SGCB), LGMD2F (Gene: SGCD), LGMD2G (Gene: TCAP), LGMD2H (Gene: TRIM32), LGMD2I (Gene: FKRP), LGMD2J (Gene: TTN), LGMD2K (Gene: POMT1), LGMD2L (Gene: ANO5), LGMD2M (Gene: FKTN), LGMD2N (Gene: POMT2), LGMD2O (Gene: POMGNT1), LGMD2Q (Gene: PLEC1)), Congenital muscular dystrophy, Distal muscular dystrophy (Subtypes and affected genes: Miyoshi myopathy (Gene: DYSF), Distal myopathy with anterior tibial onset (Gene: DYSF), Welander distal myopathy (Gene: TIA1), Gowers-Laing distal myopathy (Gene: MYH7), Nonaka distal myopathy, hereditary inclusion-body myositis type 1, distal myopathy with vocal cord and pharyngeal weakness, ZASP-related myopathy), Facioscapulohumeral muscular dystrophy (Subtypes and affected genes: Type 1 (Gene: DUX4), Type 2 (Gene: SMCHD1)), Oculopharyngeal muscular dystrophy (affected gene: PABPN1), and/or myotonic dystrophy (Subtypes and affected genes: DM1 (Gene: DMPK) and DM2 (Gene: ZNF9)).

Preferably, the myotonia is congenital myotonia (affected gene: CLCN1; subtypes: Type Thomsen, Type Becker) and/or Paramyotonia congenital (affected gene: SCN4A).

Preferred types of cardiomyopathy are hypertrophic cardiomyopathy, arrhythmogenic right ventricular dysplasia, dilated cardiomyopathy, restrictive cardiomyopathy, left ventricular noncompaction, Takotsubo cardiomyopathy, myocarditis, eosinophilic myocarditis, and ischemic cardiomyopathy. Preferably, the hypertrophic cardiomyopathy is CMH1 (Gene: MYH7), CMH2 (Gene: TNNT2), CMH3 (Gene: TPM1), CMH4 (Gene: MYBPC3), CMH5, CMH6 (Gene: PRKAG2), CMH7 (Gene: TNNI3), CMH8 (Gene: MYL3), CMH9 (Gene: TTN), CMH10 (Gene: MYL2), CMH11 (Gene: ACTC1), or CMH12 (Gene: CSRP3). Preferably, the arrhythmogenic right ventricular dysplasia is ARVD1 (Gene: TGFB3), ARVD2 (Gene: RYR2), ARVD3 , ARVD4 , ARVD5 (Gene: TMEM43), ARVD6, ARVD7 (Gene: DES), ARVD8 (Gene: DSP), ARVD9 (Gene: PKP2), ARVD10 (Gene: DSG2), ARVD11 (Gene: DSC2), or ARVD12 (Gene: JUP).

Preferably, the muscular disease is Duchenne muscular dystrophy, a myotubular myopathy (Gene: MTM1), Glycogen storage disease type II (Pompe disease, Gene: GAA), or a cardiomyopathy.

Also preferably, in case the subject is a horse, the muscular disease is Hyperkalemic periodic paralysis disease (Gene: HYPP); also preferably, in case the subject is a dog, the muscular disease is canine x-linked muscular dystrophy CXMD (gene: DMD).

The term "muscle regeneration", as used herein, relates to induction of muscle cell or muscle regeneration by a means or method of the present invention, mediating an at least temporary increase of muscle mass compared to the untreated state. Thus, muscle regeneration also includes, preferably, the generation of muscle mass exceeding the amount of muscle mass exceeding the previously existing muscle mass. Preferably, said increase of muscle mass lasts for at least 1 month, more preferably at least 6 months, still more preferably at least one year, most preferably at least two years. Also encompassed by the term muscle regeneration is a normalization or re-establishment of muscle structure after pathological deterioration. Preferably, said increase of muscle mass or improvement of muscle structure is statistically significant. As used herein, muscle regeneration may be therapeutic, preferably as part of a treatment as specified herein above, or may be non-therapeutic, preferably e.g. as cosmetic muscle regeneration in human or performance increase or increase of weight gain in animals.

Preferably, the AAV capsid polypeptide is associated with a pharmaceutically active compound. The term "active compound" is, in principle understood by the skilled person.

Preferably, the term relates to a compound which, when administered to a subject, preferably a muscle cell of a subject, causes or contributes to a detectable change in said subject and/or muscle cell. Preferably, said detectable change is a change in gene expression, epigenetic, genomic, proteomic and/or metabolomic composition of said subject and/or muscle cell. Preferably, the active compound is a "pharmaceutically active compound", i.e. is a compound causing or contributing to treatment and/or prevention of a disease or disorder referred to herein. Also preferably, the active compound is a non-therapeutically active compound and/or is an active compound used at concentrations not mediating treatment effect or preventive effects and/or is administered such that no treatment effect or preventive effect is obtained.

Preferred active compounds are polypeptides or polynucleotides. Preferably, the polypeptide is a nuclease, more preferably a Cas nuclease; appropriate nucleases, in particular Cas nucleases are well known in the art. Preferably, the active compound, more preferably the pharmaceutically active compound, is a polynucleotide. Preferably, the polynucleotide comprises a non-disease mediating variant of a mutated gene in a subject causing the muscular disease or a fragment thereof; preferably, said non-disease mediating variant is comprised in an expressible polypeptide and, preferably, mediates expression of a non-disease mediating variant of the disease causing gene. Also preferably, said non-disease mediating variant mediates, preferably in conjunction with a nuclease, exchange a said disease mediating variant in the genome of a subject for a said non-disease mediating variant. Also preferably, the polynucleotide comprises a nucleic acid sequence reducing or abolishing expression of a disease mediating variant of a gene from the genome of a subject. Thus, preferably, the polynucleotide comprises an expressible construct mediating expression of an siRNA, miRNA, ribozyme or similar molecular tool known to the skilled person, said non-disease mediating variant, reducing or abolishing expression of a disease mediating variant of a gene from the genome of a subject. Preferred genes mediating muscular disease are those described herein above in the context of the respective muscular diseases. Also preferably, the polynucleotide being an active compound comprises at least one expressible construct for at least one factor for in vivo reprogramming into full or partial pluripotency, preferably for at least one of the Yamanaka factors (Takahashi & Yamanaka (2006); Lazaro (2017)) Oct-3/4, Klf4, Sox2, and c-Myc; preferably, the coding sequence for said factor or factors encoded is or are from the same species as the subject or are heterologous sequences which were codon-adapted to the subject.

The term "associated" with an active compound, as used herein, relates to an association permitting the AAV capsid polynucleotide and the active compound to enter a host cell under appropriate conditions, preferably in vivo. Preferably, said association is a bonding with the K_{d} value as specified herein above. Accordingly, association preferably includes covalent binding of the active compound to the AAV capsid polypeptide.

It is, however, also envisaged that the active compound is comprised in an AA-viral particle or an AA-virus like particle. The term "adeno-associated virus like particle" or "AA-VLP", as used herein, refers to a viral particle derived from an AAV capable of infecting a host cell, but not replicating within said host cell. Preferably, AA-VLPs have an essentially typical AAV structure as analyzed by electron microscopy, i.e. they have a capsid; however, in contrast to the normal AAV particle, the AA-VLPs of the present invention do not contain a polynucleotide capable of directing generation of viral progeny. In an embodiment, the AA-VLPs are empty, i.e. they do not contain AAV DNA, preferably no DNA at all. Preferably, the AA-VLPs comprise the AAV proteins known to the skilled artisan to be comprised in an AAV particle, preferably at least comprise the AAV capsid polypeptide as specified herein above, more preferably consist of the AAV capsid polypeptide as specified herein above. As will be understood by the skilled person, in case the active compound is a polynucleotide, the polynucleotide preferably comprises packaging signals required for packaging the polynucleotide into an AAV particle or VLP. In a further embodiment, the polynucleotide being the active compound is comprised in an otherwise replication competent AAV genome. As will be understood by the skilled person, the aforesaid modes of associating active compounds may also be combined, e.g. by providing a guide RNA for a Cas nuclease on a polynucleotide packaged into an AAV particle or AA-VLP, and associating a Cas nuclease, e.g. by covalent binding, to said particle.

Advantageously, it was found in the work underlying the present invention that the AAV capsid polypeptides proposed herein mediate an improved tropism of AAV particles to muscle tissue and muscle cells, while at the same time exhibiting a strongly reduced liver tropism. Thus, AAV capsids comprising said AAV capsid polypeptides are improved vehicles for delivering active compounds to muscle cells, in particular upon systemic application.

The definitions made above apply mutatis mutandis to the following. Additional definitions and explanations made further below also apply for all embodiments described in this specification mutatis mutandis.

The present invention further relates to a polynucleotide encoding the AAV capsid polypeptide of the present invention for use in treating and/or preventing a muscular disease and/or in muscle regeneration.

The term "polynucleotide", as used herein, relates to a polynucleotide comprising a nucleic acid sequence which encodes an AAV capsid polypeptide as specified herein above, the AAV capsid polypeptide having the biological activity as described herein above. Preferably, the polynucleotide comprises, more preferably consists of, the nucleic acid sequence of SEQ ID NO:13, 15, 17, 19, 21, 23, 25, 27, 29, 31, 33, or 35. It is to be understood that a polypeptide having an amino acid sequence as detailed above may also be encoded due to the degenerated genetic code by more than one species of polynucleotide. Moreover, the term "polynucleotide" as used in accordance with the present invention further encompasses variants of the aforementioned specific polynucleotides. Said variants may represent orthologs, paralogs or other homologs of the polynucleotide of the present invention. The polynucleotide variants, preferably, comprise a nucleic acid sequence characterized in that the sequence can be derived from the aforementioned specific nucleic acid sequences by at least one nucleotide substitution, addition and/or deletion whereby the variant nucleic acid sequence shall still encode an AAV capsid polypeptide having the activity as specified above. Variants also encompass polynucleotides comprising a nucleic acid sequence which is capable of hybridizing to the aforementioned specific nucleic acid sequences, preferably, under stringent hybridization conditions. These stringent conditions are known to the skilled worker and can be found in standard text books. A preferred example for stringent hybridization conditions are hybridization conditions in 6x sodium chloride/sodium citrate (= SSC) at approximately 45°C, followed by one or more wash steps in 0.2x SSC, 0.1% SDS at 50°C to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of 0.1 to 5x SSC (pH 7.2). If organic solvent is present in the abovementioned buffer, for example 50% formamide, the temperature under standard conditions is approximately 42°C. The hybridization conditions for DNA:DNA hybrids are preferably for example 0.1x SSC and 20°C to 45°C, preferably between 30°C and 45°C. The hybridization conditions for DNA:RNA hybrids are preferably, for example, 0.1x SSC and 30°C to 55°C, preferably between 45°C and 55°C. The abovementioned hybridization temperatures are determined for example for a nucleic acid with approximately 100 bp (= base pairs) in length and a G + C content of 50% in the absence of formamide. The skilled worker knows how to determine the hybridization conditions required by referring to textbooks. Alternatively, polynucleotide variants are obtainable by PCR-based techniques such as mixed oligonucleotide primer-based amplification of DNA, i.e. using degenerated primers against conserved domains of the polypeptides of the present invention. Conserved domains of the polypeptides of the present invention may be identified by a sequence comparison of the nucleic acid sequence of the polynucleotide or of the amino acid sequence of the polypeptides as specified above. Suitable PCR conditions are well known in the art. As a template, DNA or cDNA from AAVs may be used. Further, variants include polynucleotides comprising nucleic acid sequences which are at least 70%, at least 75%, at least 80%, at least 85%, at least 90%, at least 95%, at least 98% or at least 99% identical to the nucleic acid sequences detailed above. Moreover, also encompassed are polynucleotides which comprise nucleic acid sequences encoding amino acid sequences which are at least 70%, at least 75%, at least 80%, at least 85%, at least 90%, at least 95%, at least 98% or at least 99% identical to the amino acid sequences referred to above. The percent identity values are, preferably, calculated over the entire amino acid or nucleic acid sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (J. Mol. Evolution., 25, 351-360, 1987, Higgins et al., CABIOS, 5 1989: 151-153) or the programs Gap and BestFit (Needleman and Wunsch (J. Mol. Biol. 48; 443-453 (1970)) and Smith and Waterman (Adv. Appl. Math. 2; 482-489 (1981)), which are part of the GCG software packet (Genetics Computer Group, 575 Science Drive, Madison, Wisconsin, USA 53711 (1991)), are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments. A polynucleotide comprising a fragment of any of the aforementioned nucleic acid sequences is also encompassed as a polynucleotide. The fragment shall encode a polypeptide which still has the biological activity as specified above. Accordingly, the polypeptide may comprise or consist of the domains of the polypeptide of the present invention conferring the said biological activity. A fragment as meant herein, preferably, comprises at least 50, at least 100, at least 250 or at least 500 consecutive nucleotides of any one of the aforementioned nucleic acid sequences or encodes an amino acid sequence comprising at least 20, at least 30, at least 50, at least 80, at least 100 or at least 150 consecutive amino acids of any one of the aforementioned amino acid sequences. The polynucleotides either essentially consist of the aforementioned nucleic acid sequences or comprise the aforementioned nucleic acid sequences. Thus, they may contain further nucleic acid sequences as well. Specifically, the polynucleotides of the present invention may encode fusion proteins wherein one partner of the fusion protein is a polypeptide being encoded by a nucleic acid sequence recited above. Such fusion proteins may, preferably, comprise as additional part polypeptides for monitoring expression (e.g., green, yellow, blue or red fluorescent proteins, alkaline phosphatase and the like) or so-called "tags" which may serve as a detectable marker or as an auxiliary measure for purification purposes. Tags for the different purposes are well known in the art and comprise FLAG-tags, 6-histidine-tags, MYC-tags and the like. The polynucleotide shall be provided, preferably, either as an isolated polynucleotide (i.e. isolated from its natural context) or in genetically modified form. The polynucleotide, preferably, is DNA, including cDNA, or RNA. The term encompasses single- as well as double-stranded polynucleotides. Moreover, preferably comprised are also chemically modified polynucleotides including naturally occurring modified polynucleotides such as glycosylated or methylated polynucleotides or artificially modified ones such as biotinylated polynucleotides.

The present invention also relates to a vector comprising the polynucleotide according to the present invention for use in treating and/or preventing a muscular disease and/or in muscle regeneration.

The term "vector", preferably, encompasses phage, plasmid, viral or retroviral vectors as well as artificial chromosomes, such as bacterial or yeast artificial chromosomes. More preferably, vector relates to a vector derived from an AAV. Moreover, the term also relates to targeting constructs which allow for random or site-directed integration of the targeting construct into genomic DNA. Such targeting constructs, preferably, comprise DNA of sufficient length for either homologous or heterologous recombination. The vector encompassing the polynucleotides as specified herein, preferably, further comprises selectable markers for propagation and/or selection in a host. The vector may be incorporated into a host cell by various techniques well known in the art. For example, a plasmid vector can be introduced in a precipitate such as a calcium phosphate precipitate or rubidium chloride precipitate, or in a complex with a charged lipid or in carbon-based clusters, such as fullerens. Alternatively, a plasmid vector may be introduced by heat shock or electroporation techniques. Should the vector be a virus, it may be packaged in vitro using an appropriate packaging cell line prior to application to host cells. Viral vectors may be replication competent or replication defective. In the latter case, viral propagation generally will occur only in complementing host/cells. More preferably, in the vector, the polynucleotide is operatively linked to expression control sequences allowing expression in prokaryotic or eukaryotic cells or isolated fractions thereof. Expression of said polynucleotide comprises transcription of the polynucleotide, preferably into a translatable mRNA. Regulatory elements ensuring expression in eukaryotic cells, preferably mammalian cells, are well known in the art. They, preferably, comprise regulatory sequences ensuring initiation of transcription and, optionally, poly-A signals ensuring termination of transcription and stabilization of the transcript. Additional regulatory elements may include transcriptional as well as translational enhancers. Possible regulatory elements permitting expression in prokaryotic host cells comprise, e.g., the *lac*, *trp* or *tac* promoter in *E. coli*, and examples for regulatory elements permitting expression in eukaryotic host cells are the *AOX1* or *GAL1* promoter in yeast or the CMV-, SV40-, RSV-promoter (Rous sarcoma virus), CMV-enhancer, SV40-enhancer or a globin intron in mammalian and other animal cells. Moreover, inducible expression control sequences may be used in an expression vector. Such inducible vectors may, preferably, comprise *tet* or *lac* operator sequences or sequences inducible by heat shock or other environmental factors. Suitable expression control sequences are well known in the art. Beside elements which are responsible for the initiation of transcription such regulatory elements may also comprise transcription termination signals, such as the SV40-poly-A site or the tk-poly-A site, downstream of the polynucleotide. In this context, suitable expression vectors are known in the art such as Okayama-Berg cDNA expression vector pcDV1 (Pharmacia), pBluescript (Stratagene), pCDM8, pRc/CMV, pcDNA1, pcDNA3 (Invitrogen) or pSPORT1 (GIBCO BRL). Expression vectors derived from viruses such as retroviruses, vaccinia virus, adeno-associated virus, herpes viruses, or bovine papilloma virus, may be used for delivery of the polynucleotides or vector of the invention into targeted cell population. Methods used to construct recombinant viral vectors are well known to those skilled from standard text books. Preferably, the vector is an AAV vector, more preferably a self-complementary AAV vector, as described in e.g. Grimm et al. (2006).

The present invention also relates to a host cell comprising the AAV capsid polypeptide according to the present invention, the polynucleotide according to the present invention, and/or the vector according to the present invention for use in treating and/or preventing a muscular disease and/or in muscle regeneration.

As used in this specification, the term "host cell" preferably relates to a bacterial cell, a yeast cell, or an insect cell, or a mammalian cell. Preferably the host cell is a mammalian cell. Also preferably, the host cell is a muscle cell, more preferably a smooth muscle cell or a striated muscle cell. Most preferably, the cell is a striated muscle cell. Preferably, the host cell is an in vivo cell, i.e. a cell comprised in a living subject. More preferably, the host cell is a cell maintained in vitro, preferably in a suitable cultivation medium. Preferably, the host cell is capable of producing AAV capsid polypeptides.

Furthermore, the present invention relates to an adeno-associated virus (AAV) capsid comprising an AAV capsid polypeptide according to the present invention for use in treating and/or preventing a muscular disease and/or in muscle regeneration.

The term "AAV capsid" is understood by the skilled person; components of the AAV capsid are known in the art. Preferably, the AAV capsid does not comprise a per se replication competent AAV genome; preferably, the AAV capsid is an AA-VLP as specified herein above. The AAV capsid comprising an AAV capsid polypeptide comprises at least one AAV capsid polypeptide as specified herein, i.e. an AAV capsid polypeptide bonded to a binding peptide as specified herein; accordingly, the other AAV capsid polypeptides constituting the AAV capsid may be different AAV capsid polypeptides. Preferably, the AAV capsid polypeptides according to the present invention and the further AAV capsid polypeptides comprised in an AAV capsid are derived from the same AAV strain. Preferably, the AAV capsid comprises at least ten, more preferably at least 20, even more preferably at least 30, still more preferably at least 40, still more preferably, at least 50 AAV capsid polypeptides according to the present invention. Most preferably, the AAV capsid comprises AAV capsid polypeptides according to the present invention as the only capsid polypeptides, i.e., preferably, as the only VP polypeptides. Preferably, the AAV capsid consists of AAV capsid polypeptides according to the present invention. As will be understood, the AAV capsid may comprise more than one type of AAV capsid polypeptide according to the present invention, e.g. may comprise one or more AAV capsid polypeptides comprising SEQ ID NO:2 and one or more AAV capsid polypeptides comprising SEQ ID NO:3. More preferably, however, the AAV capsid comprises one type of AAV capsid polypeptide according to the present invention, more preferably consists of one type of AAV capsid polypeptide, said AAV capsid polypeptide being an AAV capsid polypeptide according to the present invention. Preferably, the AAV capsid further comprises at least one pharmaceutically active agent as specified elsewhere herein.

The present invention also relates to a pharmaceutical composition comprising an AAV capsid polypeptide according to the present invention, a polynucleotide according to the present invention, a vector according to the present invention, a host cell according to the present invention, and/or an AAV capsid according to the present invention for use in treating and/or preventing a muscular disease and/or in muscle regeneration.

The term "composition", as used herein, relates to a mixture of compounds comprising at least an AAV capsid polypeptide, a polynucleotide, a vector, and/or an AAV capsid as specified herein and at least one carrier. The carrier(s) must be acceptable in the sense of being compatible with the other ingredients of the composition and being not deleterious to a potential recipient thereof. The carrier employed may be, for example, a gel or a liquid. Exemplary of liquid carriers are phosphate-buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax. Suitable carriers comprise those mentioned above and others well known in the art. The carrier(s) is/are selected so as not to affect the biological activity of the composition. Examples of such diluents are distilled water, physiological saline, Ringer's solutions, dextrose solution, and Hank's solution. Preferably, in the composition, at least 50%, preferably at least 75%, more preferably at least 90%, even more preferably at least 95%, most preferably essentially all viral particles are viral particles comprising a polynucleotide as specified elsewhere herein.

As is understood by the skilled person, the composition, in particular the pharmaceutical composition, may comprise further AAV capsids and/or vectors, preferably according to the invention. E.g., a composition may comprise further AAV capsids and/or vectors providing a different tropism for auxiliary treatment (e.g. co-treatment of a muscle and its exciting nerve), or AAV capsids and/or vectors comprising the same AAV capsid polypeptide(s), but carrying different active compounds (Grimm et al. (2010)).

The composition according to the present specification is a pharmaceutical composition; thus, preferably, the carrier is a pharmaceutically acceptable carrier. In addition, the pharmaceutical composition or formulation may also include other carriers, adjuvants, or nontoxic, nontherapeutic, non-immunogenic stabilizers and the like. The pharmaceutical compositions are, preferably, administered topically or systemically. Suitable routes of administration conventionally used for drug administration are topical, intravenous, or parenteral administration as well as inhalation. Preferably, administration is systemic, more preferably intravenous. However, depending on the nature and mode of action of a compound, the pharmaceutical compositions may be administered by other routes as well, in particular as specified elsewhere herein. Moreover, the polynucleotide can be administered in combination with other drugs either in a common pharmaceutical composition or as separated pharmaceutical compositions wherein said separated pharmaceutical compositions may be provided in form of a kit of parts. The binding polypeptide is, preferably, administered in conventional dosage forms prepared by combining the drugs with standard pharmaceutical carriers according to conventional procedures. These procedures may involve mixing or dissolving the ingredients as appropriate to the desired preparation. It will be appreciated that the form and character of the pharmaceutically acceptable carrier or diluent is dictated by the amount of active ingredient with which it is to be combined, the route of administration and other well-known variables.

A therapeutically effective dose refers to an amount of the polynucleotide to be used in a pharmaceutical composition of the present invention which prevents, ameliorates or treats the symptoms accompanying a disease or condition referred to in this specification. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. The dosage regimen will be determined by the attending physician and other clinical factors; preferably in accordance with any one of the above described methods. As is well known in the medical arts, dosages for any one patient depend upon many factors, which may include the patient's size, body surface area, age, the particular compound to be administered, sex, time and route of administration, general health, and other drugs being administered concurrently. Progress can be monitored by periodic assessment. A typical dose can be, for example, in the range of 1 to 1000 µg; however, doses below or above this exemplary range are envisioned, especially considering the aforementioned factors. Generally, the regimen as a regular administration of the pharmaceutical composition should be in the range of 1 µg to 10 mg units per day. If the regimen is a continuous infusion, it should also be in the range of 1 µg to 1 mg units per kilogram of body weight per minute, respectively. Preferably, the pharmaceutical composition is administered once to the subject, i.e., preferably, is used as a one-time treatment. Thus, preferably, treating or preventing a disease or condition with a compound recited in this specification consists of a single administration of said compound. Depending on the subject and the mode of administration, the quantity of substance administration may vary over a wide range to provide from about 0.01 mg per kg body mass to about 10 mg per kg body mass. As will be appreciated, in case a vector is used, e.g. an AAV vector, quantity of substance administration may be even lower, e.g. from 1 ng to 1 mg per dose, preferably as a one-time dose. Preferably, in case the compound of the present invention is administered packaged in a viral vector, the dose is of from 10¹⁰ to 10¹⁶ viral particles per administration. The pharmaceutical compositions and formulations referred to herein are administered at least once in order to treat or ameliorate or prevent a disease or condition recited in this specification. However, the said pharmaceutical compositions may be administered more than one time, for example from one to four times. Progress can be monitored by periodic assessment.

Specific pharmaceutical compositions are prepared in a manner well known in the pharmaceutical art and comprise at least one active compound referred to herein above in admixture or otherwise associated with a pharmaceutically acceptable carrier or diluent. For making those specific pharmaceutical compositions, the active compound(s) will usually be mixed with a carrier or the diluent. The resulting formulations are to be adapted to the mode of administration, i.e. in the forms of tablets, capsules, suppositories, solutions, suspensions or the like. Dosage recommendations shall be indicated in the prescriber's or user's instructions in order to anticipate dose adjustments depending on the considered recipient.

The present invention also relates to a method for treating and/or preventing a muscular disease and/or for muscle regeneration comprising contacting a subject with an AAV capsid polypeptide according to the present invention, a polynucleotide according to the present invention, a vector according to the present invention, a host cell according to the present invention, and/or an AAV capsid according to the present invention.

The method for treating and/or preventing and/or for muscle regeneration of the present invention, preferably, is an in vivo method. Moreover, it may comprise steps in addition to those explicitly mentioned above. For example, further steps may relate, e.g., to providing an AAV capsid polypeptide, a polynucleotide, a vector, and/or an AAV capsid according to the present invention, or further treatment/prevention steps before, concomitantly, or after said contacting. Moreover, one or more of said steps may be performed by automated equipment.

The present invention also relates to a use of an AAV capsid polypeptide according to the present invention, a polynucleotide according to the present invention, a vector according to the present invention, a host cell according to the present invention, and/or an AAV capsid according to the present invention for transferring an active compound into a muscle cell.

The use for transferring an active compound into a muscle cell of the present invention, preferably, is an in vitro use. It is, however, also envisaged that the use, preferably, is an in vivo use; in such case, more preferably, the use is non-therapeutic, e.g. cosmetic. Preferably, said use is for induction of muscle cell or muscle regeneration in the field of plastic modification of the whole body, especially the face and the upper body including the bottom.

Moreover, the present invention relates to a use of an AAV capsid polypeptide according to the present invention, a polynucleotide according to the present invention, a vector according to the present invention, a host cell according to the present invention, and/or an AAV capsid according to the present invention for the manufacture of a medicament for the treatment of a muscular disease.

In view of the above, the following embodiments are particularly preferred:
All references cited in this specification are herewith incorporated by reference with respect to their entire disclosure content and the disclosure content specifically mentioned in this specification.

### Figure Legends

Fig. 1: Tissue specificity of muscle variants. The normalized proportion per cell of AAV9_P1, AAV9wt, AAVpolwt, AAVpol_A1 and AAV6wt is shown in the analyzed tissues. Abbreviations: abdominal aorta (Aa), thoracic aorta (At), brain (B), blood cells (B1C), colon (C), diaphragm (Di), duodenum (Du), eye (Eye), brown fat (FatB), white fat (FatW), heart (H), inner ear (I), kidney (K), liver (Li), lung (Lu), ovaries (O), pancreas (P), quadriceps femoris (QF), spleen (S) and stomach (St).
Fig. 2: Efficiency comparison in muscle tissues. Normalized proportions are shown for the top 15 variants in the diaphragm, heart and quadriceps femoris. Abbreviations as in Fig. 1.
Fig. 3: Tissue specificity of muscle variants. The normalized proportion per cell of AAV9_P1, AAV9wt, AAV6wt, AAVpolwt, AAVpol_A1 and AAV9_P3 is shown in the analyzed tissues. Abbreviations as in Fig. 1.
Fig. 4: Tissue specificity of muscle variants. The normalized proportion per cell of AAVM41, AAVB1, AAV2_MTP, AAV9_K3 and AAV9LD is shown in the analyzed tissues. Abbreviations as in Fig. 1.
Fig. 5: Efficiency of AAV9_P1 in muscle tissues. The bar plots show the normalized proportion of the top 15 AAV variants in the diaphragm, heart, biceps and quadriceps femoris. Abbreviations as in Fig. 1.
Fig. 6: cDNA analysis of muscle-specific variants. Shows EYFP relative quantities of AAV9_P1, AAVpol_A1, AAVpolwt, AAV9wt and uninjected control. Depicted values are the mean of three mice with SD. For AAV9wt one mouse had to be excluded due to no viral transcripts in none of the analyzed tissues.
Fig. 7: Overview for the peptide insertion site of muscle variants illustrating the surrounding amino acids of the peptide insertion. The flanking amino acids for AAV9_P1, AAV9_P3, AAVpol_A1 and AAV9_K3 were changed to enable a SfiI-mediated peptide insertion cloning.

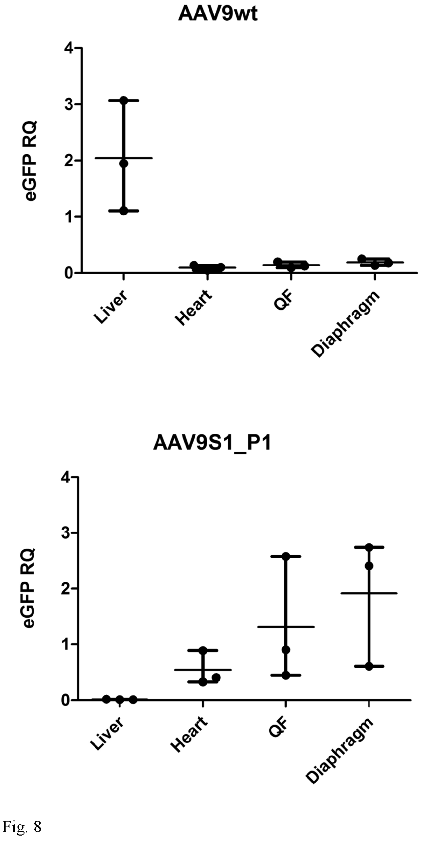

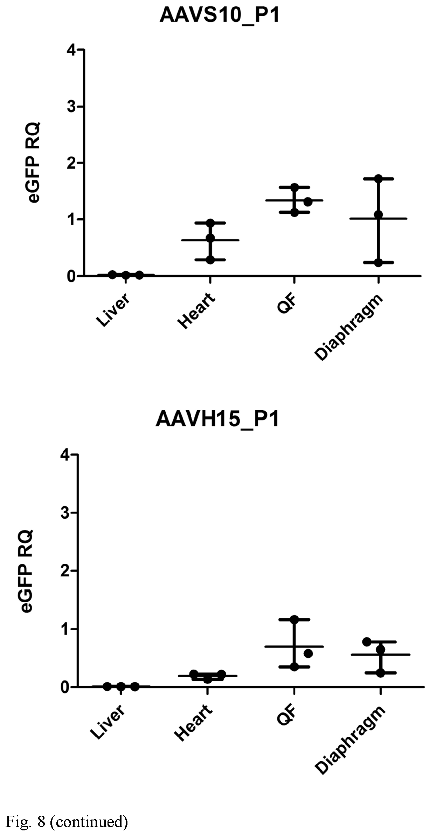

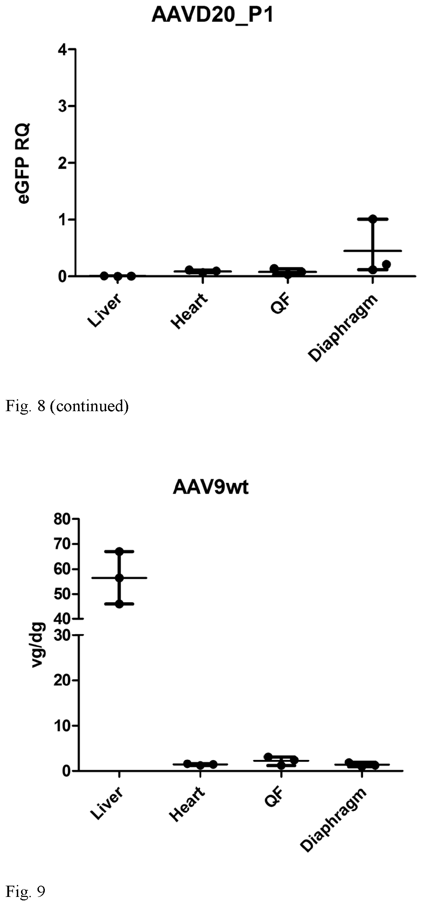

Fig. 8: cDNA analysis of muscle-specific variants. Shown are eGFP relative quantifications (RQ=2-ΔCT of eGFP and RNA polII) as determined by qPCR. Depicted values are the mean of three mice with range for each of the following variants: AAV9wt, AAVS1_P1, AAVS10_P1, AAVH15_P1, and AAVD20_P1. Mice were injected with 2.5x10¹¹ vg of the corresponding AAV variant. QF: quadriceps femoris.

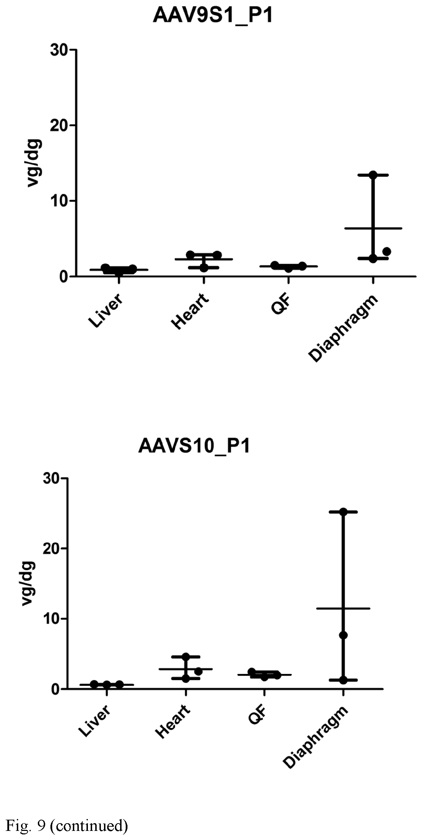

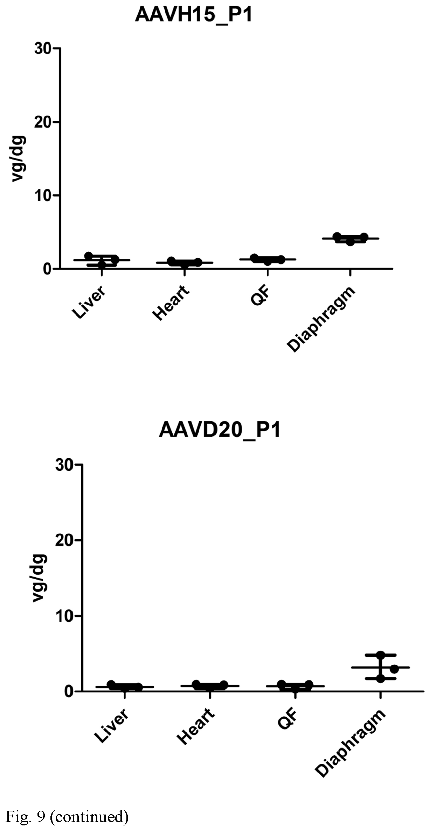

Fig. 9: Genomic (g)DNA analysis of muscle-specific variants. Shown are viral genomes per diploid genome (vg/dg) values as determined by ddPCR of eGFP and RPP30. Depicted values are the mean of three mice with range for each of the following variants: AAV9wt, AAVS1_P1, AAVS10_P1, AAVH15_P1, and AAVD20_P1. Mice were injected with 2.5x10¹¹ vg of the corresponding AAV variant. QF: quadriceps femoris.

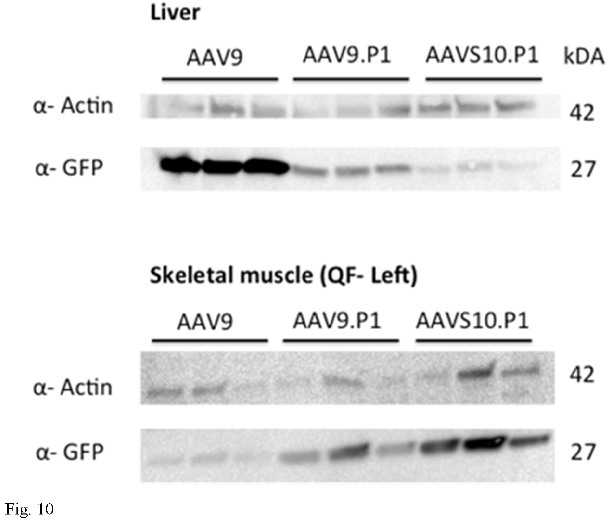

Fig. 10: Comparison of eGFP expression (reporter) mediated by AAVS10_P1 versus AAV9wt and AAV9_P1 in liver and skeletal muscle (quadriceps femoris) by employing immunoblotting. Each lane is a representation of one mouse (3 mice per variant) injected with equal dose (2.5x10¹¹ viral genomes) into the tail vain. AAVS10_P1 shows enhanced de-targeting from liver and increased expression in skeletal muscle in comparison with AAV9 and AAV9_P1. Total protein was quantified using BCA (Bicinchonic acid) protein assay to ensure equal loading of protein samples extracted from the corresponding tissues.
Fig. 11: Sequence logos of the sequence space enriched from a randomized AAV9 library in skeletal muscle, heart, and diaphragm after one (A) and two (B) rounds of selection.
Fig. 12: cDNA analysis of muscle-specific variants as in Fig. 8, with more organs analyzed for AAV9wt (A) compared to AAV9S1_P1 (B) and AAV9S10_P1 (C); (D) Relative biodistribution of the indicated AAV variants in liver compared to AAV9.

The following Examples shall merely illustrate the invention. They shall not be construed, whatsoever, to limit the scope of the invention.

### Example 1: Methods

For this study a self-complementary AAV genome was used carrying a CMV promoter-driven EYFP transgene flanked by a BGH (bovine growth hormone) poly-A signal. A 15 nt-long barcode is positioned in the 3-prime UTR and will therefore be transcribed allowing the tracking of the corresponding AAV on the RNA and DNA level. During AAV production, HEK293T cells are transfected by an Adenoviral helper plasmid delivering essential genes for the replication of the AAV. Additionally, a plasmid for the expression of rep2 (rep gene of AAV2) and the respective cap gene is needed as well as a plasmid carrying the ITR-flanked (inverted terminal repeats), barcoded AAV genome. By selecting capsid/barcode pairs for the production, a packaging of a defined barcoded genome into a synthetic capsid was allowed. Each AAV variant was subsequently purified with an iodixanol gradient and viral titers were determined. Equimolar amounts of all variants were pooled to generate a barcoded AAV library, comprising AAV9_P1 and important benchmark variants (Varadi et al. (2012), Yu et al. (2009), Choudhury et al. (2016), Yang et al. (2009), and Adachi et al. (2014)).

For the biodistribution evaluation of the library, six female C57BL/6 mice were injected with 1x10¹² vg (vector genomes) per mouse via the tail vein. After one week, abdominal aorta (Aa), thoracic aorta (At), brain (B), blood cells (B1C), colon (C), diaphragm (Di), duodenum (Du), eye (Eye), brown fat (FatB), white fat (FatW), heart (H), inner ear (I), kidney (K), liver (Li), lung (Lu), ovaries (O), pancreas (P), quadriceps femoris (QF), spleen (S) and stomach (St) were harvested. Subsequent DNA and RNA isolation was performed to enable, firstly, cDNA synthesis and, secondly, barcode enrichment by PCR. The following next generation sequencing determined the proportion of each variant in every tissue of the six mice based on the occurrence of the barcode sequence. Furthermore q(uantitative)PCRs for the detection of viral genomes in the individual organs provided all necessary values for the multi-step normalization procedure which allows a comparison between different organs for the same AAV variant. Final results were acquired by using a tailored Python script (modified from Marsic et al., 2015).

### Example 2: Results

### 2.1 First screening with AAV9_P1

The collected data allowed a tissue specificity analysis of one variant over all tissues as well as an efficiency analysis of all variants within one tissue.

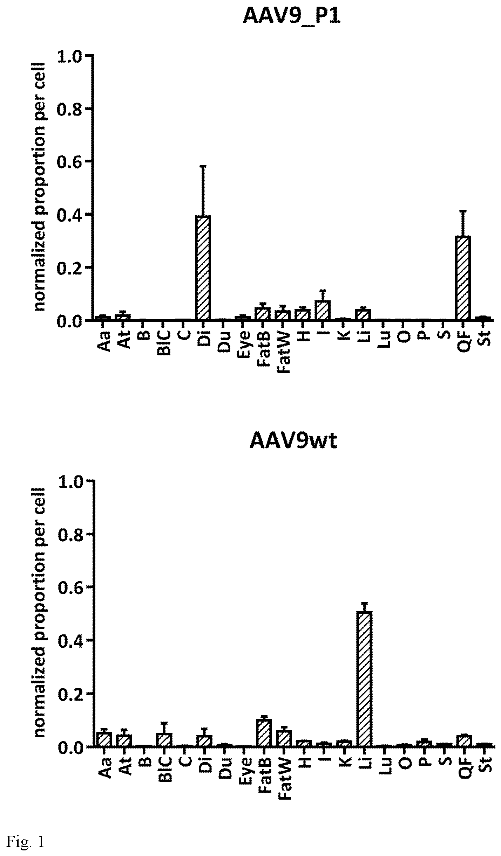

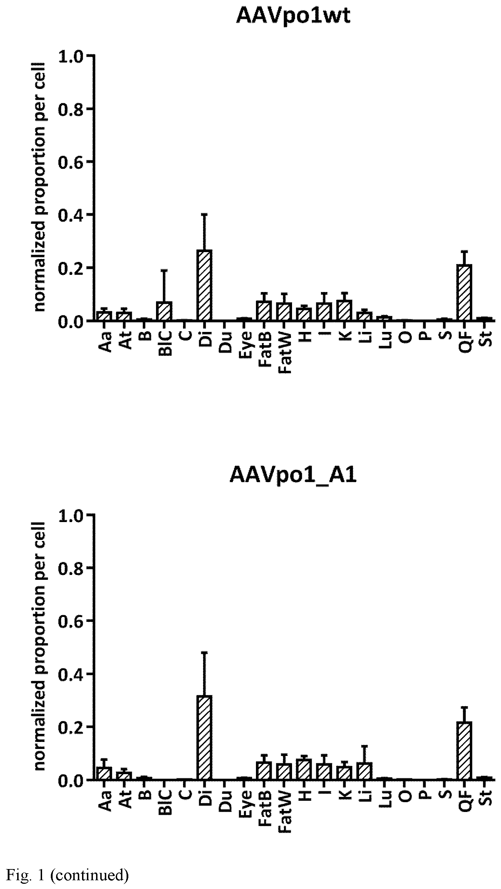

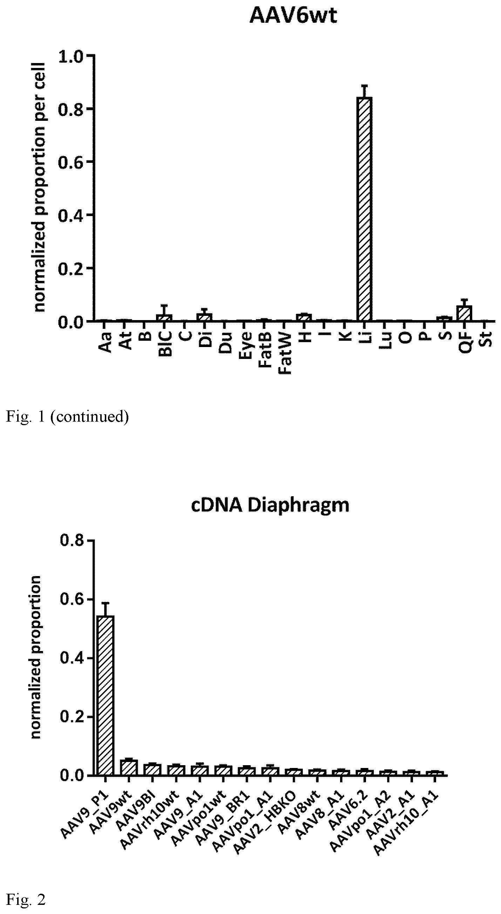

Regarding organ specificity (Figure 1 and Table 2), AAV9_P1 demonstrated a strong muscle tropism with 74% of the virus ending up in the analyzed muscle tissues diaphragm, heart and quadriceps femoris. In contrast, when using the parental virus AAV9wt, only 10% of corresponding transcripts were found in the muscle tissues, whereas 50% could be observed in the major off-target of AAV, the liver. Based on these data, a 13-fold reduction was detected in the liver for AAV9_P1. With AAV6wt, another well-known AAV for muscle targeting, again only 10% of the total viral transcripts could be found in the muscles versus 84% in the liver. Previously identified muscle-specific candidates, AAVpolwt and AAVpol_A1, showed a strong muscle specificity with 51% and 60%, respectively, and minor off-targeting in the liver (3% and 6%, respectively).

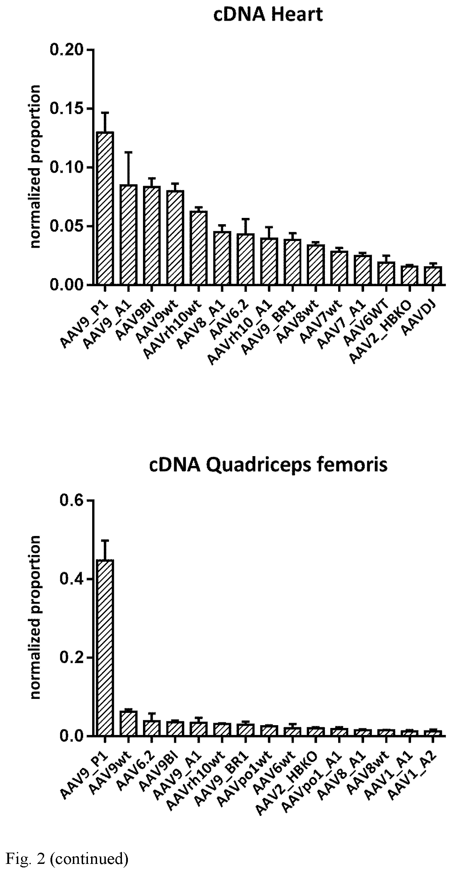

A direct comparison of the efficiency of the capsids in the muscle tissues illustrates the superiority of AAV9_P1 (Figure 2). Out of all viral transcripts expressed from the 82 variants in the library, 54%, 45% and 13% belong to AAV9_P1 in the diaphragm, quadriceps femoris and heart, respectively. A comparison with the benchmarks revealed major improvements in terms of entering the target tissue and transcribing the barcoded transgene (Table 1), as well as muscle specificity (Table 2).

**Table 1: AAV9_P1 efficiency comparison. AAV9_P1 demonstrates major efficiency improvements over other AAV capsids that are currently considered as benchmarks for muscle delivery. Values depicted are fold-changes of AAV9_P1 transduction as compared to the indicated variants.**

| Variant | Diaphragm | Heart | Quadriceps f. |
|---|---|---|---|
| AAV9wt | 10.6 | 1.6 | 7.2 |
| AAVpo1wt | 18.0 | 10.8 | 18.6 |
| AAVpo1_A1 | 21.6 | 9.2 | 25.5 |
| AAV6wt | 78.4 | 6.8 | 22.4 |

**Table 2: AAV9_P1 specificity comparison. Analyzed was the muscle specificity of AAV9_P1, AAV9wt, AAVpolwt, AAVpo1_A1 and AAV6wt. Depicted values are percentages of detected transcripts compared to all analyzed tissues.**

| Variant | Muscle tissues [%] | Liver [%] |
|---|---|---|
| AAV9_P1 | 74.6 | 3.8 |
| AAV9wt | 10.0 | 50.3 |
| AAVpo1wt | 51.7 | 2.9 |
| AAVpo1_A1 | 60.3 | 6.2 |
| AAV6wt | 10.3 | 83.9 |

### 2.2 Second screening with AAV9_P1

To solidify the enhanced muscle specificity and efficiency of AAV9_P1, another NGS-based screening was carried out following the exact same workflow as described in the first screening but now also including three published AAV variants reported to be superior in muscle tissue, AAVM41 (Yang et al. (2009)), AAVB1 (Choudhury et al. (2016)) and AAV2_MTP (Yu et al. (2009)). Furthermore two additional peptide insertion variants were tested, AAV9_P3 and AAV9_K3 (Varadi et al. (2012)), which carry a similar peptide motif like AAV9_P1, offering the possibility to understand the role of the peptide itself for the improved performance in muscle.

For generation of the second barcoded AAV library, the first library was expanded by adding 75 additional variants including the benchmark variants. Six female C57BL/6 mice were injected with 1.57x10¹² vg/mouse via the tail vein and kept for one week before harvesting the aorta (A), biceps (Bi), colon (C), diaphragm (Di), duodenum (Du), eye (Eye), brown fat tissue (FatB), white fat tissue (FatW), heart (H), inner ear (I), kidney (K), liver (Li), lung (Lu), ovaries (O), pancreas (P), quadriceps femoris (QF) and stomach (St). From the spleen and the lymph nodes, CD3+, CD19+, CD11b+ and CD11c+ cells were isolated by MACS to study the ability of AAVs to enter immune cells. RNA and DNA was extracted from all tissues, and samples were processed and prepared for the NGS-based analysis. The data revealed clear outliers for mouse 3 and 4 for AAV9_P1; therefore, both were excluded. The following graphs show the mean values of mouse 1, 2, 5 and 6 and the corresponding SD.

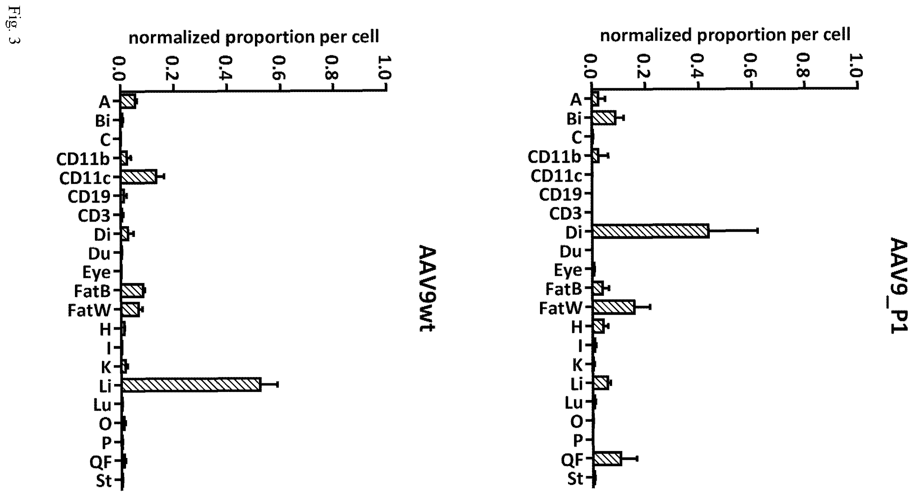

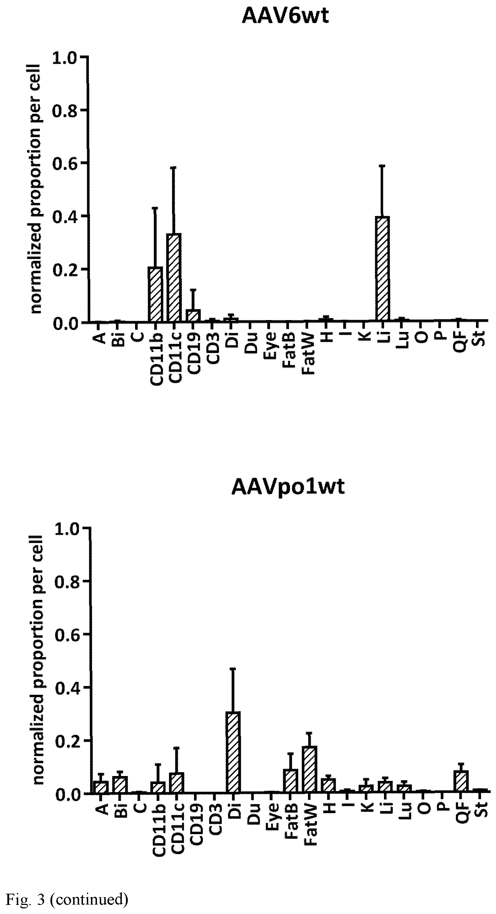

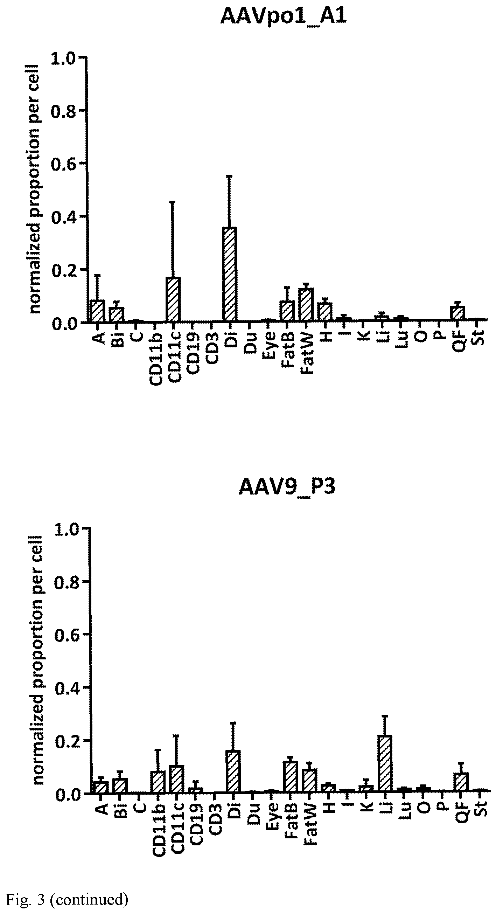

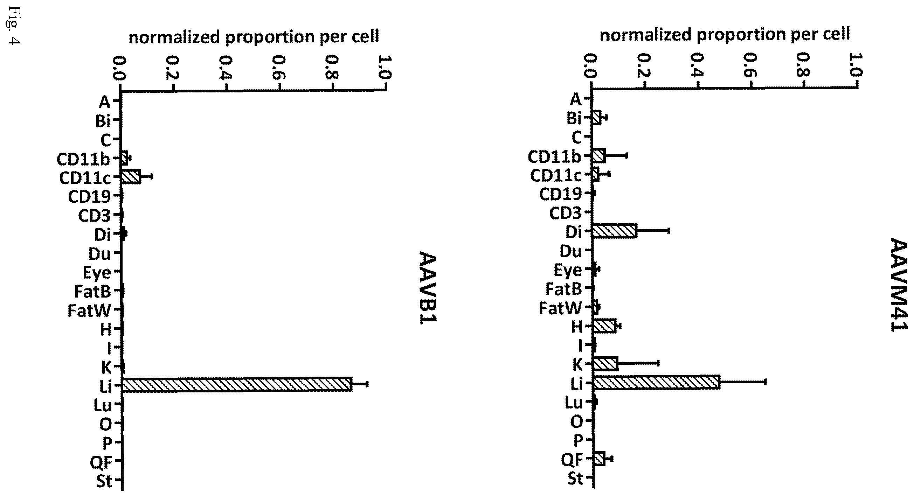

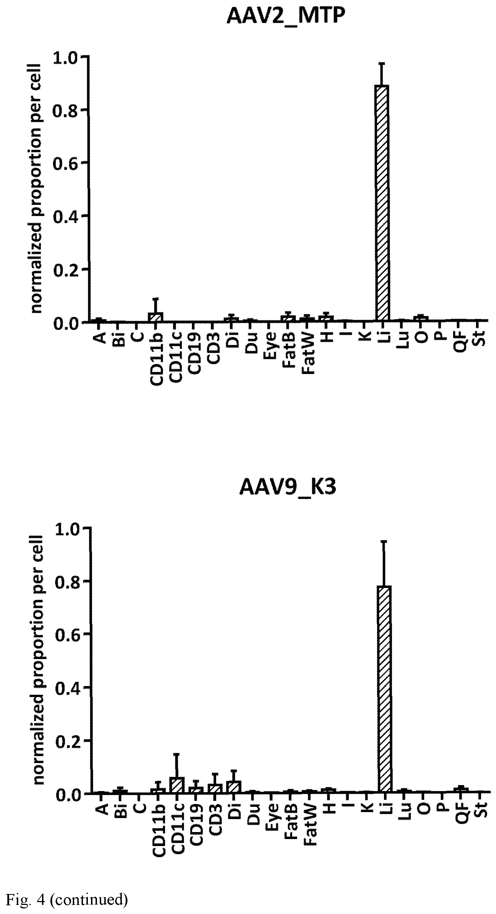

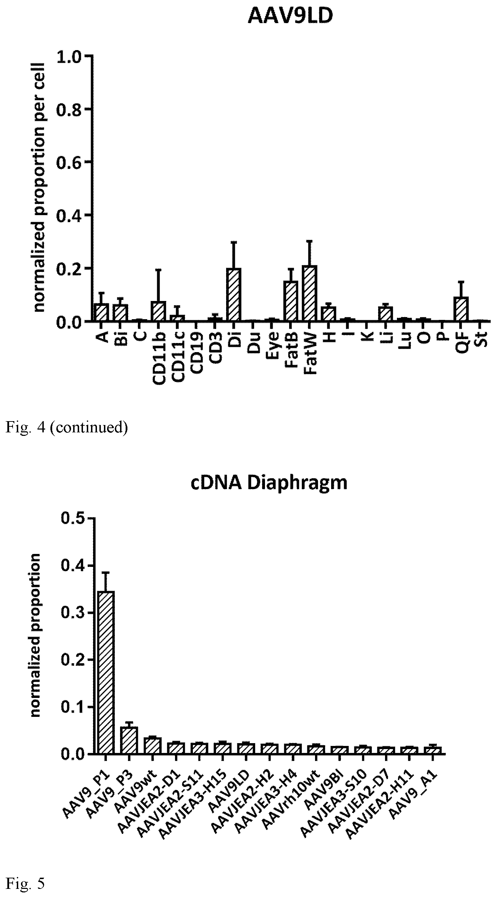

As observed previously in the first screening, AAV9_P1 demonstrated an enhanced muscle tropism (66%) as well as detargeting from the liver (5.5%) (Figure 3). Regarding the wild-type AAVs, only AAVpolwt displays inherent muscle specificity. The published benchmark variants for muscle transduction, AAVM41, AAVB1 and AAV2_MTP, are clearly inferior to AAV9_P1 (Figure 4). Only AAVM41 shows a tendency to preferably transduce the four analyzed muscle tissues. AAVB1 and AAV2_MTP predominantly end up in the liver. Particularly informative is a comparison of AAV9_P1 to AAV9_K3 and AAV9_P3. AAV9_K3, for instance, differs in only two amino acids as compared to the P1 peptide but is inserted at position 589, whereas P1 was integrated at position 588. While these seem to be minor changes, the biodistribution analysis of AAV9_K3 reveals a strong tropism for the liver which even exceeds the one seen for AAV9wt. The peptide motif of AAV9_P3 varies in four amino acids positions but this time a muscle tropism can be seen though to a weaker extent than with AAV9_P1 (Figure 3). Interestingly, AAV9LD, a variant with two point mutations as compared to AAV9wt, is massively detargeted from the liver as originally reported (Adachi et al., 2014). However, at the same time, it is enriched in muscle tissues (Figure 4).

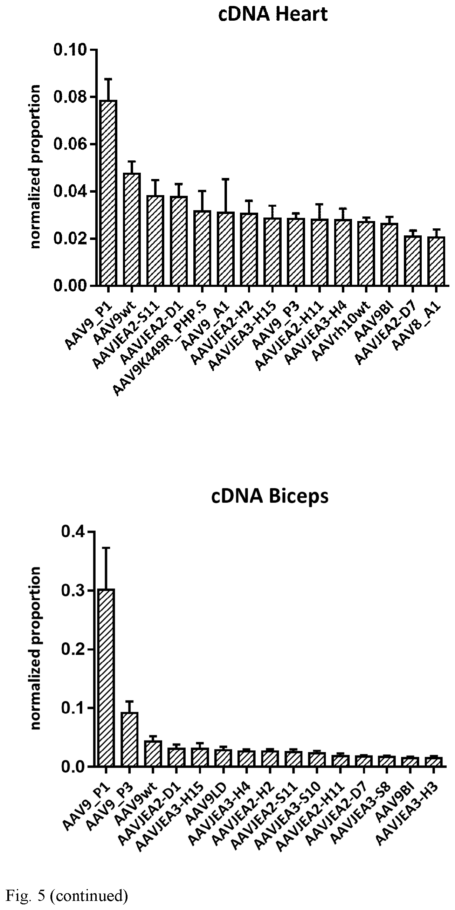

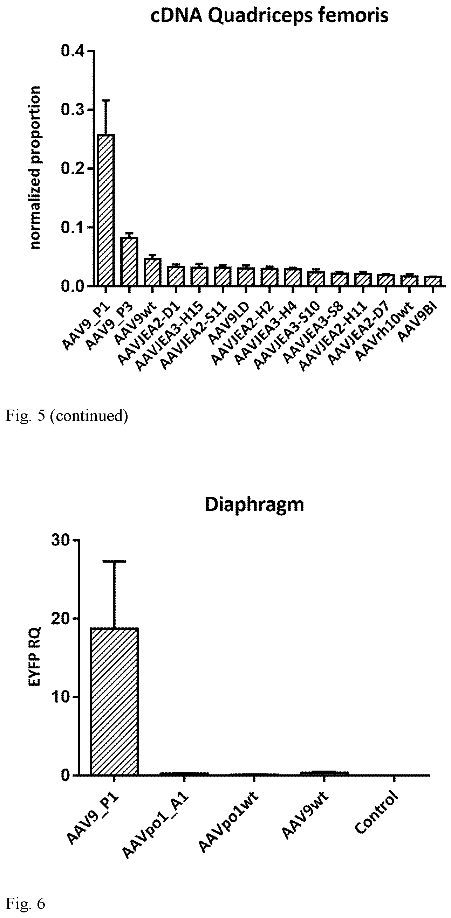

An analysis of the proportions of each variant within one tissue gave similar results as in the first screening (Figure 5). AAV9_P1 is the most efficient vector in this study in the diaphragm, biceps, quadriceps femoris and, to a lesser extent, in the heart. A more detailed overview of the differences is seen in Tables 3 and 4.

**Table 1: AAV9_P1 efficiency comparison. Efficiency of AAV9_P1 in comparison to AAV9_P3, AAV9wt, AAV9LD, AAV9_K3, AAVB1, AAVpolwt, AAVpol_A1, AAVM41, AAV2_MTP and AAV6wt. Values are fold-differences of AAV9_P1 transduction as compared to the indicated variants.**

| Variant | Diaphragm | Heart | Quadriceps f. | Biceps |
|---|---|---|---|---|
| AAV9_P3 | 6.1 | 2.8 | 3.1 | 3.3 |
| AAV9wt | 10.1 | 1.6 | 5.5 | 7.0 |
| AAV9LD | 16.1 | 5.0 | 8.5 | 10.5 |
| AAV9_K3 | 31.3 | 6.0 | 21.4 | 21.0 |
| AAVB1 | 35.6 | 4.9 | 42.3 | 64.5 |
| AAVpo1wt | 36.7 | 17.5 | 26.8 | 30.5 |
| AAVpo1_A1 | 46.1 | 14.5 | 38.9 | 42.3 |
| AAVM41 | 109.9 | 14.8 | 75.7 | 80.7 |
| AAV2_MTP | 543.2 | 63.6 | 1135.4 | 1409.5 |
| AAV6wt | 248.2 | 16.1 | 146.5 | 195.6 |

**Table 2: AAV9_P1 specificity comparison. Muscle specificity of AAV9_P1, AAV9_P3, AAV9wt, AAV9LD, AAV9_K3, AAVB1, AAVpo1wt, AAVpo1_A1, AAVM41, AAV2_MTP and AAV6wt. Depicted values are percentages of detected transcripts as compared to all analyzed tissues.**

| Variant | Muscle tissues [%] | Liver [%] |
|---|---|---|
| AAV9_P1 | 66.6 | 5.5 |
| AAV9_P3 | 29.6 | 20.8 |
| AAV9wt | 5.3 | 52.0 |
| AAV9LD | 39.4 | 5.2 |
| AAV9_K3 | 7.5 | 77.3 |
| AAVB1 | 1.4 | 86.1 |
| AAVpo1wt | 48.4 | 3.6 |
| AAVpo1_A1 | 51.5 | 1.5 |
| AAVM41 | 31.7 | 47.3 |
| AAV2_MTP | 2.9 | 88.3 |
| AAV6wt | 2.2 | 38.8 |

### Example 3: Muscle validation of AAV9_P1

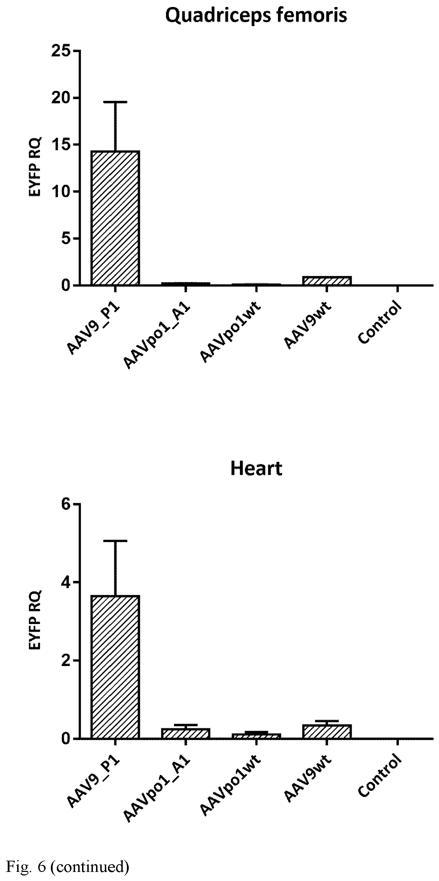

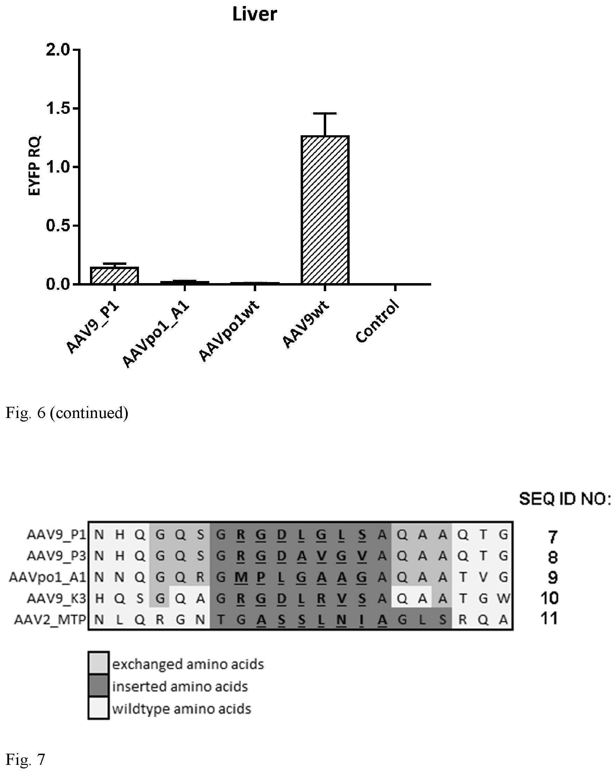

To confirm the muscle specificity of AAV9_P1, a follow-up study was conducted using female C57BL/6 mice which were injected with 1x10¹¹vg/mouse via the tail vein. Besides AAV9_P1, the lead candidates from a previous screening, AAVpo1wt and AAVpo1_A1, were used as well as the in muscle tissue highly efficient serotype, AAV9wt. This time, all AAV variants were injected individually into three mice per group. Potential interfering effects originating from receptor competition in a library context are thereby eliminated, allowing to evaluate AAV9_P1 performance. Mice were kept for one week after injection before diaphragm, heart, liver and quadriceps femoris were collected for qPCR analysis against the viral transcripts, using POLR2A as a housekeeper (Figure 6).

The evaluation of EYFP relative quantities for AAV9_P1 showed a superior increase compared to AAV9wt in diaphragm (55-fold), quadriceps femoris (17-fold) and heart (11-fold) while being 9-fold less abundant in the liver. In addition the peptide insertion variant outcompetes AAVpo1wt and AAVpol_A1 in all muscle tissues. On the other hand both porcine variants exhibit lower cDNA levels in the liver which is however not enough to outweigh their major disadvantage in terms of efficiency.

### Example 4: In vivo enrichment of targeting sequences

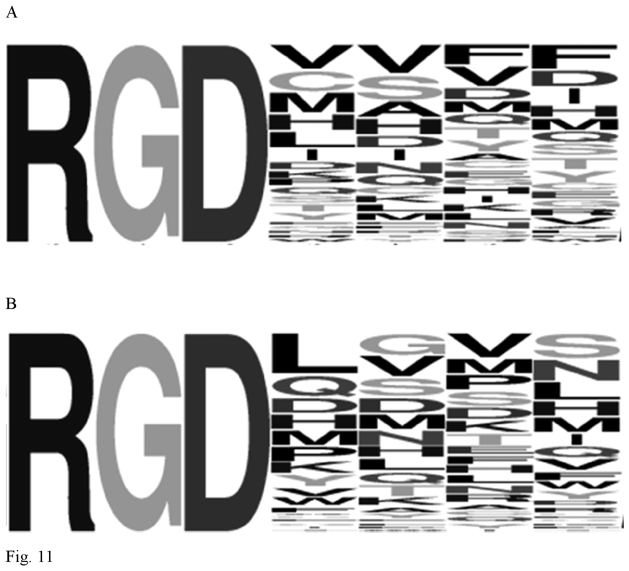

An AAV9-library was constructed encoding capsid polypeptides with the sequence of SEQ ID NO:1 inserted between amino acids 588 and 589, in which positions X¹ to X⁴ were completely randomized and in which the sequence was flanked by the amino acids S/RG N-terminally and A C-terminally. Virus particles comprising the library were administered into mice via the tail vein, and re-isolated from skeletal muscle, heart, and diaphragm. After the first round of selection, a sequence space represented by the sequence logo of Fig. 11A was obtained, while after the second round of selection, the sequence space represented by the sequence logo of Fig. 11B was retrieved, showing an enrichment of specific amino acids.

### Example 5: Tissue tropism and biodistribution of AAV variants

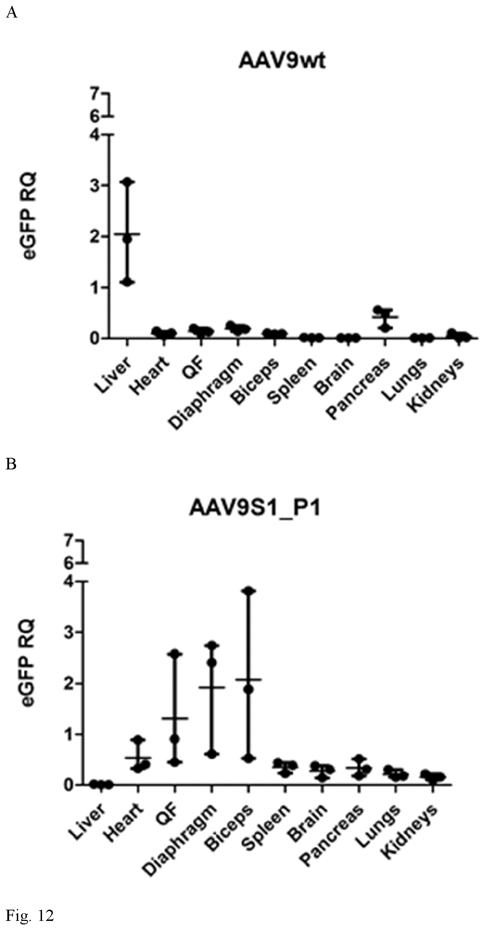

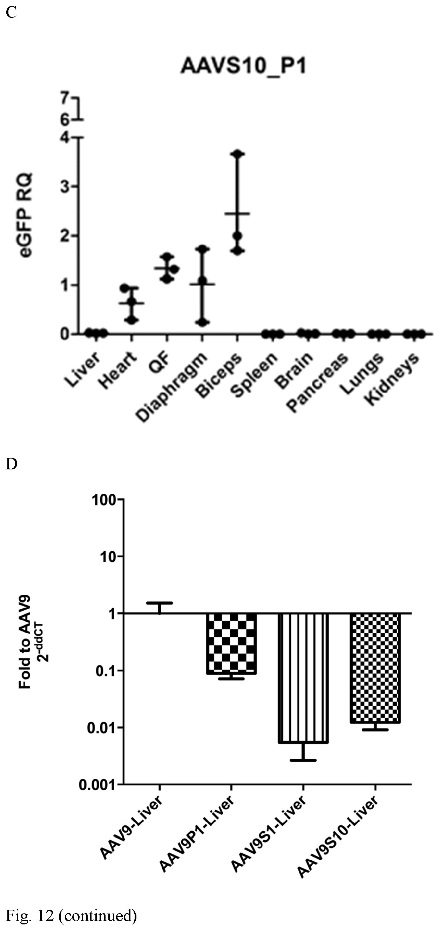

The tissue tropism of AAV9S1_P1 (having a capsid with the amino acid sequence of SEQ ID NO:30) and AAVS10_P1 (having a capsid with the amino acid sequence of SEQ ID NO:28) was compared to AAV9wt as described herein above in Example 1 for further organs, using 2.5E11 vg per mouse; results are shown in Fig. 12A-C. Further, biodistribution of AAV9P1, AAV9S1, and AAV9S10 in liver was compared to that of AAV9 as described above in Example 1; results are shown in Fig. 12D.

Non-standard literature cited:
- Adachi et al. (2014), Nat. Commun., vol. 5, no. June 2011, p. 3075.
- Choudhury et al. (2016), Mol. Ther., vol. 24, no. 7, pp. 1247-1257.
- Grimm et al. (2006), Nature 441, 537-541.
- Grimm et al. (2010), J. Clin. Invest. 120, 3106-3119.
- Kienle, PhD Thesis, Ruprecht-Karls-Universität Heidelberg, 2014.
- Kunze et al. (2018), Glia, vol. 66, no. 2, pp. 413-427.
- Lazaro et al. (2017). bioRxiv doi: 10.1101/101188.
- Marsic et al. (2015), Mol. Ther. Methods Clin. Dev., vol. 2, no. September, p. 15041.
- Michelfelder et al. (2009), PLOS One 4(4):e5122
- Takahashi & Yamanaka (2006), Cell 126, 663-676.
- Varadi et al. (2012), Gene Ther., vol. 19, no. 8, pp. 800-809.
- Yang et al. (2009), Proc. Natl. Acad. Sci. U. S. A., vol. 106, no. 10, pp. 3946-51.
- Yu et al. (2009), Gene Ther., vol. 16, no. 8, pp. 953-962.

## Claims

1. An adeno-associated virus (AAV) capsid polypeptide bonded to a binding peptide comprising an amino acid sequence RGDLGLS (SEQ ID NO:2), wherein said binding peptide is inserted into the amino acid sequence of said AAV capsid polypeptide and wherein the AAV capsid polypeptide with the inserted binding peptide comprises, preferably consists of, an amino acid sequence as shown in SEQ ID NO:28 or 30, or an amino acid sequence at least 98% identical to SEQ ID NO:28 or 30.

2. The AAV capsid polypeptide of claim 1, wherein said AAV capsid polypeptide with the inserted binding peptide comprises, more preferably consists of, the amino acid sequence of SEQ ID NO:28 or 30 or a sequence at least 99% identical to SEQ ID NO:28 or 30.

3. The AAV capsid polypeptide of claim 1 or 2, wherein said AAV capsid polypeptide with the inserted binding peptide comprises, preferably consists of, an amino acid sequence as shown in SEQ ID NO:28 or 30.

4. A polynucleotide encoding the AAV capsid polypeptide as specified in any one of claims 1 to 3.

5. The polynucleotide of claim 4, wherein said polynucleotide comprises, preferably consists of, a nucleic acid sequence as shown in SEQ ID NO:29 or 31 or a nucleic acid sequence at least 70% identical to SEQ ID NO:29 or 31.

6. The polynucleotide of claim 4 or 5, wherein said polynucleotide comprises, preferably consists of, a nucleic acid sequence as shown in SEQ ID NO:29 or 31.

7. An AAV capsid comprising an AAV capsid polypeptide as specified in any one of claims 1 to 3.

8. The AAV capsid of claim 7 for use in medicine.

9. The AAV capsid of claim 7 for use in transferring a pharmaceutically active compound into a muscle cell.

10. In vitro use of the AAV capsid of claim 7 for transferring a non-therapeutically active compound into a muscle cell.

11. The AAV capsid for use of claim 9 or the in vitro use of claim 10, wherein said active compound is a polynucleotide.

12. The AAV capsid for use of claim 9 or 11 or the in vitro use of claim 10 or 11, wherein said muscle is a striated muscle, preferably heart or a skeletal muscle or diaphragm.

13. The AAV capsid polypeptide of any one of claims 1 to 3 or the AAV capsid of claim 7 for use in treating and/or preventing a muscular disease and/or in muscle regeneration.

14. The AAV capsid polypeptide for use of claim 13 or the AAV capsid for use of claim 13, wherein said muscular disease is a muscular dystrophy, a cardiomyopathy, a myotonia, a muscular atrophy, a myoclonus dystonia, a mitochondrial myopathy, a rhabdomyolysis, a fibromyalgia, and/or a myofascial pain syndrome.

15. The subject matter of any one of claims 9 to 14, wherein said muscle is a striated muscle, preferably heart or a skeletal muscle or diaphragm.
